# EUROPÄISCHE PATENTSCHRIFT

(11) **EP 0 897 290 B1**
(45) Veröffentlichungstag und Bekanntmachung des Hinweises auf die Patenterteilung: **13.11.2002**
(21) Anmeldenummer: 97915372.3
(22) Anmeldetag: 15.03.1997
(51) Int. Cl.: A61B 17/88, A61C 8/00, B25B 23/142

(54) **CHIRURGISCHER DREHMOMENTSCHLÜSSEL**
SURGICAL TORQUE WRENCH
CLE DYNAMOMETRIQUE A USAGE CHIRURGICAL

(30) Priorität: 18.04.1996 DE 19615241
(43) Veröffentlichungstag der Anmeldung: 24.02.1999
(73) Patentinhaber: Aesculap AG & Co. KG, 78532 Tuttlingen (DE)
(72) Erfinder: BEGER, Jens, D-78532 Tuttlingen (DE)
(74) Vertreter: Hoeger, Stellrecht & Partner
(86) Internationale Anmeldenummer: EP9701312
(87) Internationale Veröffentlichungsnummer: WO97039694

(56) Entgegenhaltungen:
- EP-A- 0 704 281
- DE-U- 8 705 205
- US-A- 5 337 638
- US-A- 5 507 211

## Beschreibung

Die Erfindung betrifft einen chirurgischen Drehmomentschlüssel mit einem Maulteil zur Anlage an einem Drehelement und mit einem an das Maulteil anschließenden Griffteil zum Verdrehen des Maulteils, bei dem das Maulteil und das Griffteil um eine parallel zur Drehachse des Drehelements verlaufende Schwenkachse schwenkbar miteinander verbunden sind, bei dem zwischen Maulteil und Griffteil eine Maulteil und Griffteil in einer bestimmten Winkelstellung relativ zueinander fixierende, beim Überschreiten eines bestimmten Drehmoments lösbare Halteeinrichtung vorgesehen ist und bei dem zwischen Maulteil und Griffteil ein Anschlag wirksam ist, der bei gelöster Halteeinrichtung den Schwenkwinkel des Maulteils gegenüber dem Griffteil begrenzt.

Derartige Drehmomentschlüssel werden verwendet, um beispielsweise bei orthopädischen Implantaten oder Endoprothesen Schrauben oder Muttern einzudrehen oder zu lösen.

Dabei wäre es wünschenswert, wenn mit dem Drehmomentschlüssel genau feststellbare Maximaldrehmomente auf die Drehelemente ausgeübt werden könnten, das heißt die Bedienungsperson sollte in die Lage versetzt werden, das Überschreiten eines bestimmten Drehmoments beim Ein- oder Ausdrehen zuverlässig festzustellen.

In der US-A-5,337,638 ist ein gattungsgemäßer Drehmomentenschlüssel beschrieben, bei dem beim Auftreten einer Überlast das Maulteil in einer Überlaststellung verbleibt. Es muß aus dieser Überlaststellung von der Benutzungsperson in eine Arbeitsstellung zurückverschwenkt werden. Dies kann in bestimmten Arbeitssituationen den Arbeitsablauf stören.

Es ist Aufgabe der Erfindung, einen gattungsgemäßen chirurgischen Drehmomentschlüssel so auszubilden, daß die Bedienungsperson einerseits das Überschreiten eines maximalen Drehmoments sicher feststellen kann, andererseits aber nach einem solchen Ausknicken nicht an der Weiterarbeit gehindert wird.

Diese Aufgabe wird bei einem chirurgischen Drehmomentschlüssel der eingangs beschriebenen Art erfindungsgemäß dadurch gelöst, daß zwischen Griffteil und Maulteil eine Feder wirksam ist, unter deren Einwirkung das Maulteil aus einer Überlaststellung mit gelöster Halteeinrichtung selbsttätig in die Arbeitsstellung zurückschwenkt, in der die Halteeinrichtung aktiv ist. Nach einem Ausknicken schwenkt daher das Maulteil selbsttätig wieder in die Arbeitsstellung zurück, so daß der Arbeitsvorgang unmittelbar fortgesetzt werden kann.

Günstig ist es, wenn zwischen Maulteil und Griffteil ein Anschlag wirksam ist, der bei gelöster Halteeinrichtung ein Verschwenken des Maulteils gegenüber dem Griffteil nur in einer Richtung ermöglicht. Das Ausknicken erfolgt daher nur in einer Richtung, in der entgegengesetzten Richtung kann dagegen der Drehmomentschlüssel ohne Ausknicken benutzt werden, also mit beliebig hohem Drehmoment. Dies kann beispielsweise dazu verwendet werden, um beim Eindrehen mit begrenztem Drehmoment zu arbeiten, beim Ausdrehen dagegen mit beliebig hohem. Andererseits ist es auch möglich, durch Umdrehen des Drehmomentschlüssels bewußt das Ausknicken des Drehmomentschlüssels zu vermeiden.

Vorzugsweise liegt die Schwenkachse zwischen dem Maul des Maulteils und dem hinteren Ende des Maulteils, wobei die Halteeinrichtung am hinteren Ende des Maulteils angreift. Insbesondere kann die Schwenkachse zum Maul einen wesentlich geringeren Abstand aufweisen als zum hinteren Ende, beispielsweise kann das Verhältnis 1:5 betragen.

Bei einer bevorzugten Ausführungsform ist vorgesehen, daß die Halteeinrichtung ein Formgehemme mit fester Auslösekraft ist. Hier sind dem Fachmann an sich verschiedene Möglichkeiten an die Hand gegeben, wesentlich ist bei einem Formgehemme der formschlüssige Eingriff eines Formteils in ein anderes Formteil, wobei dieser Eingriff durch eine feste Auslösekraft zu überwinden ist.

Bei einer besonders bevorzugten Ausführungsform ist vorgesehen, daß die Halteeinrichtung einen in Richtung auf die Schwenkachse verschiebbaren, federbelasteten Rastkörper umfaßt, der in eine Ausnehmung am Maulteil eintaucht und beim Überschreiten eines bestimmten Drehmoments gegen die Federbelastung aus der Ausnehmung ausgehoben wird.

Dabei ist es vorteilhaft, wenn der Rastkörper im Griffteil längsverschieblich gelagert ist.

Insbesondere kann der Rastkörper an einem im Griffteil längsverschieblichen Führungskörper gelagert sein, an dem eine am Griffteil abgestützte Feder angreift.

Bei einer bevorzugten Ausführungsform ist vorgesehen, daß das Griffteil eine zylindrische Führungshülse umfaßt, in der ein kolbenförmiger Führungskörper längsverschieblich gelagert ist und die eine Druckfeder aufnimmt.

Bei chirurgischen Drehmomentschlüsseln dieser Art ist es außerordentlich wichtig, daß die Auslösekräfte der Halteeinrichtung den einmal eingestellten Wert beibehalten. Derartige Drehmomentschlüssel müssen nach der Benutzung sterilisiert werden, und dabei könnten sich Änderungen der Reibungsverhältnisse ergeben, beispielsweise durch Entfernung eines Schmiermittels. Diese Änderung der Reibungsverhältnisse, beispielsweise des Führungskörpers und des Rastkörpers, könnten eine Veränderung der Auslösekräfte bewirken.

Um dies zu vermeiden, kann gemäß einer bevorzugten Ausführungsform vorgesehen sein, daß der Führungskörper und seine Führung eine schmiermittelfreie Kunststoff-Metall-Paarung bilden. Eine solche Materialpaarung ermöglicht ein gutes Gleitverhalten mit reproduzierbaren Reibungswerten, die auch durch die Sterilisiervorgänge nicht verändert werden.

Insbesondere kann der Führungskörper aus Polyetheretherketon und die Führung aus rostfreiem Stahl bestehen. Dieses Kunststoffmaterial und rostfreier Stahl bilden eine ideale Gleitpaarung und lassen sich hervorragend sterilisieren.

Günstig ist es auch, wenn der Führungskörper in der Führungshülse gegen eine Verdrehung um die Längsachse der Führungshülse gesichert ist, beispielsweise durch an der Führungshülse gelagerte, in Führungsschlitze des Führungskörpers eintauchende Stifte.

Grundsätzlich wäre es möglich, eine Halteeinrichtung mit einem immer gleichen Auslösewert zu verwenden. Günstig ist es aber, wenn die Federbelastung des Rastkörpers einstellbar ist, so daß dadurch die Auslösekraft des Drehmomentschlüssels verstellt werden kann.

Insbesondere kann vorgesehen sein, daß zur Einstellung der Federbelastung die Abstützung der Feder verstellbar ist.

Bei einer Ausgestaltung mit einer Führungshülse ist es dabei vorteilhaft, wenn die Führungshülse einen Abschlußstopfen aufweist, an dem ein verstellbarer Abstandshalter für eine Druckfederabstützung angeordnet ist.

Dieser Abstandshalter kann eine Stellschraube sein, die an einem in der Führungshülse längsverschieblichen Druckteller anliegt.

Insbesondere kann die Stellschraube in einer Gewindebohrung des Abschlußstopfens versenkt angeordnet sein, wobei es dann vorteilhaft ist, wenn die Gewindebohrung durch einen in diese eingesetzten Stopfen verschlossen ist. Die Stellschraube kann daher nur bei abgenommenem Stopfen verstellt werden, im Betrieb wird dieser Stopfen jedoch eingesetzt, so daß eine unbeabsichtigte Verstellung der Stellschraube und damit eine unbeabsichtigte Verstellung der Federbelastung ausgeschlossen sind. Dieser Stopfen kann ein Gewindestopfen sein.

Günstig ist es, wenn die Führungshülse seitliche Durchbrechungen aufweist, beispielsweise in Form von länglichen Fenstern. Dadurch ist es möglich, beim Sterilisieren auch den Innenraum der Führungshülse zuverlässig zu erreichen.

Um im Betrieb ein Eindringen von Körperflüssigkeiten etc. in die Führungshülse zu vermeiden und um gute Griffeigenschaften zu erhalten, kann vorgesehen sein, daß über die Führungshülse eine lösbar mit dieser verbundene Griffhülse geschoben ist.

Vorzugsweise ist die Griffhülse auf die Führungshülse aufgeschraubt.

Sie kann die Führungshülse und einen in sie eingesetzten Abschlußstopfen überdecken, wenn sie vollständig auf die Führungshülse aufgeschoben ist.

Gemäß einer bevorzugten Ausführungsform ist vorgesehen, daß die Griffhülse in ihrer vollständig auf die Führungshülse aufgeschobenen Position den Anschlag für das Griffteil bildet, der bei gelöster Halteeinrichtung ein Verschwenken des Maulteils gegenüber dem Griffteil nur in einer Richtung ermöglicht. Die Griffhülse verhindert also, daß das Maulteil gegenüber dem Griffteil in beiden Richtungen verschwenkt werden kann, nur in einer Richtung ist ein Ausknicken möglich, in der anderen Richtung hält die aufgeschraubte Griffhülse das Maulteil in der Arbeitsstellung und ermöglicht so, auch beliebig hohe Drehmomente in einer Drehrichtung auszuüben.

Gemäß einer bevorzugten Ausführungsform ist vorgesehen, daß der Rastkörper eine parallel zur Schwenkachse des Griffteils drehbare Rolle ist, insbesondere ist diese Rolle ein Kugellager. Dadurch erhält man genau definierte Reibungswerte zwischen Rastkörper und Maulteil, und dies ist wichtig für die Reproduzierbarkeit der Auslösekräfte.

Insbesondere kann vorgesehen sein, daß die Rolle ein gedichtetes Kugellager ist, das mit einem sterilisierbaren Fett gefüllt ist. Auch dies trägt dazu bei, daß das Instrument zwar einwandfrei sterilisierbar ist, daß aber beim Sterilisieren die Reibungsverhältnisse nicht verändert werden.

Günstig ist es, wenn die Ausnehmung am Maulteil eine bogenförmige Vertiefung in einer hinteren Stirnkante des Maulteils ist.

Diese Stirnkante kann gegenüber einer Tangente an einen um die Schwenkachse des Maulteils gelegten Kreis geringfügig geneigt sein, beispielsweise um einen Winkel zwischen 5 und 10°. Dies führt dazu, daß das Ausheben des Rastkörpers aus der Vertiefung in der einen Richtung schwer und in der anderen Richtung leicht möglich ist. In der schweren Richtung wird der Rastkörper ausgehoben, wenn der Drehmomentschlüssel beim Überschreiten eines bestimmten Drehmoments ausknickt, zur leichten Seite hin erfolgt ein Ausheben des Rastkörpers beim Auswechseln des Maulteils. Die geneigte Anordnung der Stirnkante führt dann auch dazu, daß der Rastkörper nach dem Ausheben aus der bogenförmigen Vertiefung durch Anlage an der Stirnkante diese noch weiter verschwenkt, das heißt nach dem Überschreiten einen Totpunkts unterstützt der Rastkörper beim Ausheben aus der Vertiefung noch das Ausschwenken des Maulteils, so daß das Auswechseln des Maulteils zusätzlich erleichtert wird.

Günstig ist es, wenn das Griffteil zwei parallele Arme aufweist, zwischen denen das Maulteil aufgenommen ist, und wenn die Schwenkachse des Maulteils am vorderen Ende der Arme angeordnet ist.

Ein die beiden Arme verbindender Steg kann bei einer bevorzugten Ausführungsform den Anschlag bilden, der bei gelöster Halteeinrichtung den Schwenkwinkel des Maulteils gegenüber dem Griffteil beschränkt.

Bei einer bevorzugten Ausführungsform ist vorgesehen, daß die Schwenklagerung des Maulteils am Griffteil lösbar ist. Dadurch ist es möglich, das Maulteil gegenüber dem Griffteil auszuwechseln, beispielsweise um ein Maulteil mit einem verschieden dimensionierten Maul einzusetzen, oder zum Zwecke der Sterilisierung nach der Benutzung.

Insbesondere kann dazu vorgesehen sein, daß am Griffteil eine Lagerwelle axial verschiebbar angeordnet ist, zwischen einer Lagerstellung, in der sie eine Lageröffnung des Maulteils durchsetzt, und einer Freigabestellung, in der sie aus der Lageröffnung herausgezogen ist.

Dabei ist es günstig, wenn die Lagerwelle unverlierbar an dem Griffteil gehalten ist.

Um dies zu erreichen, kann am Griffteil ein Vorsprung angeordnet sein, der in eine Längsnut der Lagerwelle eintaucht.

Dabei ist es vorteilhaft, wenn die Längsnut der Lagerwelle einen in Umfangsrichtung umbiegenden, schräg zur Längsrichtung der Lagerwelle verlaufenden Endbereich aufweist und wenn an der Lagerwelle ein Drehgriff angeordnet ist. Durch diese Form der Längsnut ergibt sich bei einer Drehung der Lagerwelle um ihre Längsachse zwangsläufig auch eine axiale Verschiebung, so daß durch Drehen am Drehgriff die Lagerwelle zwischen Freigabestellung und Lagerstellung verschoben werden kann.

Bei einer bevorzugten Ausführungsform ist vorgesehen, daß am Ende des schräg verlaufenden Endbereichs der Längsnut eine in Längsrichtung der Lagerwelle rückspringende, den Vorsprung am Griffteil in der Lagerstellung der Lagerwelle aufnehmende Ausnehmung in der Längsnut angeordnet ist und daß die Lagerwelle in der Lagerwelle federnd gegen den Vorsprung am Griffteil gedrückt ist. Dadurch ergibt sich eine bajonettartige Verriegelung der Lagerwelle in der Lagerstellung, der Vorsprung am Griffteil kann aus der rückspringenden Ausnehmung nur ausgehoben werden, wenn die Lagerwelle gegen die Wirkung einer Feder axial verschoben wird, das heißt zum Verdrehen der Lagerwelle und damit zur axialen Verschiebung ist es vorher notwendig, die Lagerwelle noch über die Lagerstellung hinausgehend axial einzudrücken.

Günstig ist es, wenn in der Lagerwelle ein stirnseitig aus dieser hervorstehender, federnd in diese eindrückbarer Druckkörper gelagert ist, der vorzugsweise als Kugel ausgebildet ist. Dieser Druckkörper liegt bei in die Lagerstellung eingeschobener Lagerwelle am Griffteil an und erzeugt die Federkraft, mit der die Lagerwelle gegen den Vorsprung am Maulteil geschoben wird, dadurch wird also die bajonettartige Verriegelung ermöglicht.

Zusätzlich ist es vorteilhaft, wenn der Druckkörper in der Freigabestellung der Lagerwelle in den Einschubraum des Maulteils hineinragt. Dadurch ergibt sich selbst dann, wenn die Lagerwelle in Freigabestellung verschoben ist, noch eine geringfügige Halterung für das Maulteil, da der Druckkörper noch geringfügig in die Lageröffnung des Maulteils hineinragt. Zur Entnahme des Maulteils muß der Druckkörper federnd in die Lagerwelle eingeschoben werden, das heißt es ist eine gewisse Kraft notwendig, um das Maulteil aus der Lagerposition zu entfernen beziehungsweise in diese einzusetzen. Einerseits verhindert dies, daß beim Verschieben der Lagerwelle in die Freigabestellung das Maulteil unbeabsichtigt aus dem Griffteil herausfällt, andererseits zeigt es beim Einsetzen des Maulteils dem Benutzer deutlich an, daß das Maultei richtig positioniert ist und daß in dieser Position die Lagerwelle in die Lageröffnung vollständig eingeschoben werden kann.

Die nachfolgende Beschreibung bevorzugter Ausführungsformen der Erfindung dient im Zusammenhang mit der Zeichnung der näheren Erläuterung. Es zeigen:

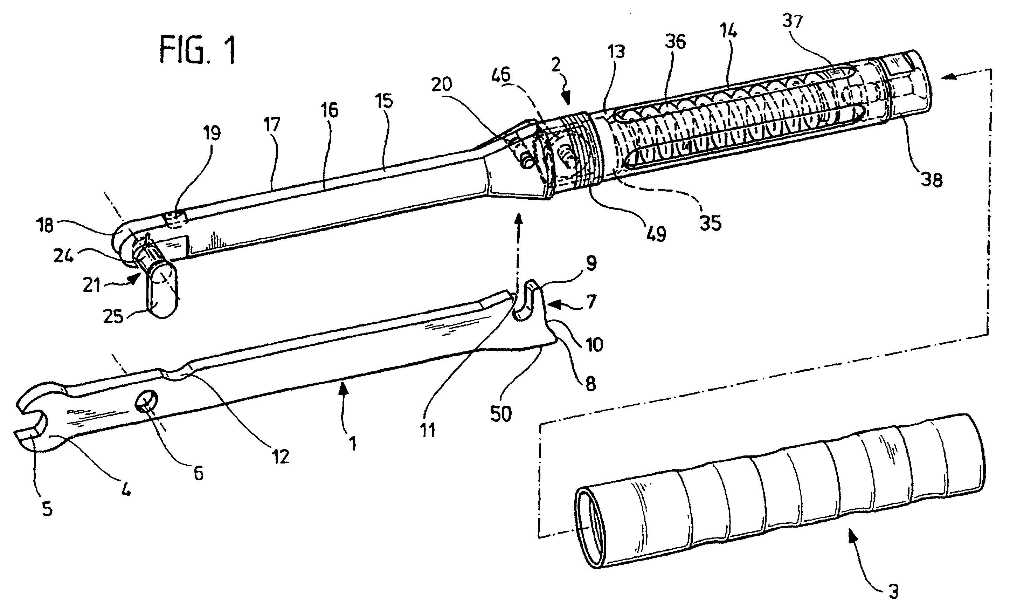

- Figur 1:: eine perspektivische Ansicht eines chirurgischen Drehmomentschlüssels im zerlegten Zustand;

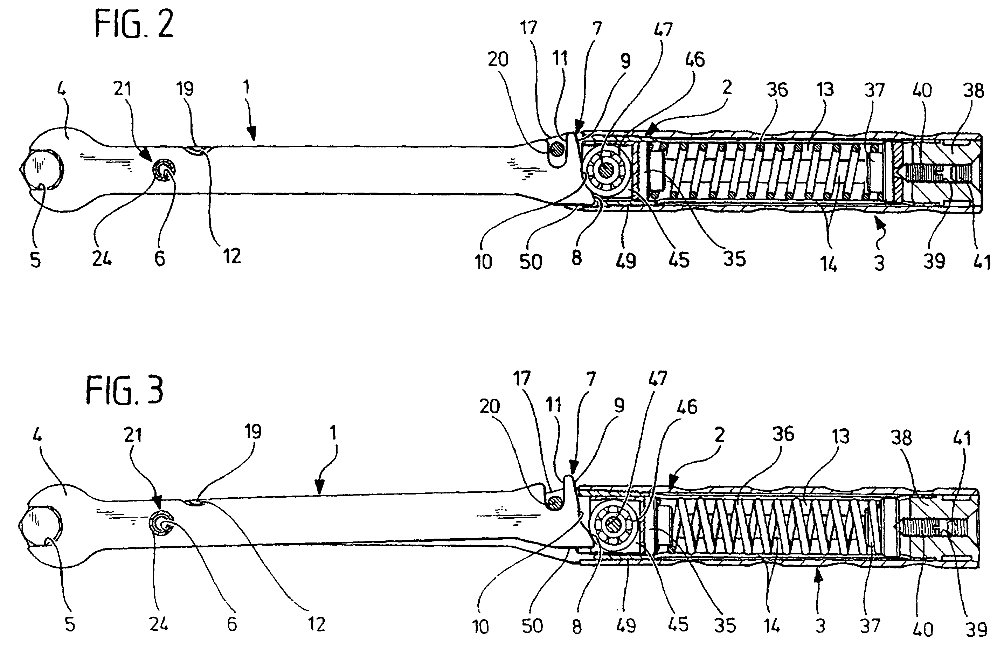

- Figur 2:: eine Längsschnittansicht des zusammengesetzten Drehmomentschlüssels der Figur 1 in Arbeitsstellung;
- Figur 3:: eine Ansicht ähnlich Figur 2 in der ausgeknickten Stellung;

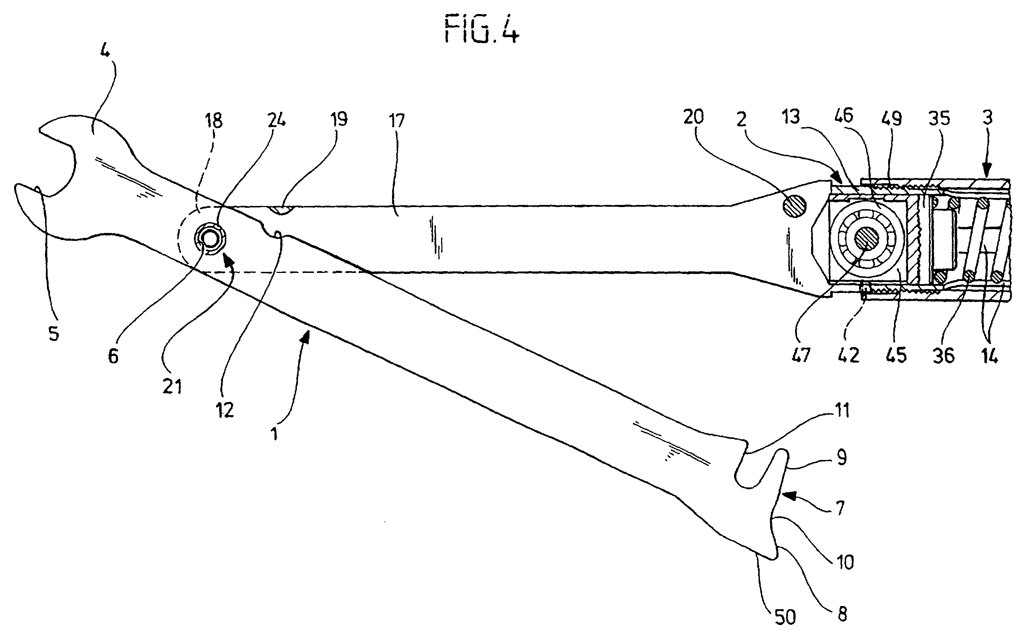

- Figur 4:: eine Längsschnittansicht des vorderen Teils des Drehmomentschlüssels der Figur 1 mit zum Auswechseln ausgeschwenktem Maulteil;
- Figur 5:: eine vergrößerte Längsschnittansicht der Halteeinrichtung des Maulteils in Arbeitsstellung;
- Figur 6:: eine Schnittansicht längs Linie 6-6 in Figur 5;
- Figur 7:: eine Draufsicht auf den Schwenklagerbereich des Maulteils am Griffteil mit eingesetztem Maulteil und mit der Lagerwelle in Lagerstellung;
- Figur 8:: eine Ansicht ähnlich Figur 7 ohne Maulteil mit der Lagerwelle in Freigabestellung;
- Figur 9:: eine Seitenansicht des Lagerbereichs der Figuren 7 und 8 mit der Lagerwelle in Lagerstellung (ausgezogene Linien) und in Freigabestellung (strichpunktierte Linien) und
- Figur 10:: eine Schnittansicht längs Linie 10-10 in Figur 7.

Der in der Zeichnung dargestellte chirurgische Drehmomentschlüssel umfaßt im wesentlichen drei Teile, nämlich ein Maulteil 1, ein Griffteil 2 und eine Griffhülse 3.

Das Maulteil 1 hat die Form eines herkömmlichen Mutternschlüssels, das heißt das Maulteil 1 hat die Form eines flachen, plattenförmigen Bandes, das am vorderen Ende einen verbreiterten Maulbereich 4 mit einem vierkantigen Maul 5 aufweist, das drehfest an eine sechskantige Mutter oder einen sechskantigen Kopf einer Schraube angelegt werden kann. In einigem Abstand neben dem Maul 5 ist im Maulteil 1 eine dieses durchsetzende Lageröffnung 6 angeordnet, der Abstand der Lageröffnung 6 vom Maul 5 ist wesentlich geringer als der Abstand der Lageröffnung 6 von der hinteren Stirnkante 7 des Maulteils 1, beispielsweise kann das Verhältnis 1:5 betragen.

Diese hintere Stirnkante 7 verläuft im wesentlichen quer zur Längsrichtung des Maulteils 1, wobei sie drei voneinander getrennte Bereiche umfaßt. Ein erster, geradliniger, an die untere Kante des Maulteils 1 anschließende Bereich 8 ist gegenüber der Längsrichtung des Maulteils 1 um einen Winkel von etwa 70 bis 75° in Richtung auf den Maulbereich 4 geneigt, ein ebenfalls geradliniger, sich an die Oberkante des Maulteils 1 anschließender Bereich 9 weist eine etwas größere Neigung gegenüber der Längsrichtung des Maulteils auf, beispielsweise in der Größenordnung von 80°. Zwischen den geradlinigen Bereichen 8 und 9 ist eine bogenförmige Vertiefung 10 angeordnet.

Unmittelbar anschließend an die Stirnkante 7 ist im Maulteil 1 von der oberen Kante desselben ausgehend ein länglicher, quer zur Längsrichtung des Maulteils 1 sich erstreckender Ausschnitt 11 vorgesehen, der fast bis zur Mitte des Maulteils 1 reicht.

Im Bereich der Lageröffnung 6 schließlich ist in der oberen Kante des Maulteils 1 ein bogenförmiger Ausschnitt 12 geringer Tiefe angeordnet.

Das Griffteil 2 umfaßt eine rohrförmige, längliche Führungshülse 13 mit länglichen, fensterförmigen Durchbrechungen 14, die an ihrem vorderen Ende zwei parallele, sich in Längsrichtung der Führungshülse 13 erstreckende, zwischen sich einen Zwischenraum 15 ausbildende Arme 16 und 17 trägt, die im Bereich ihres vorderen Endes 18 durch einen an der Oberseite der Arme 16 und 17 angeformten Steg 19 und im Übergangsbereich zur Führungshülse 13 durch einen Querstift 20 miteinander verbunden sind.

Im Bereich des vorderen Endes 18 bilden die beiden Arme 16 und 17 eine Schwenklagerung 21 für das Maulteil 1 aus. Dazu ist in einem Arm 16 eine in den Zwischenraum 15 führende Querbohrung 22 angeordnet und im anderen Arm 17 eine mit der Querbohrung 22 ausgerichtete, sich zum Zwischenraum 15 hin öffnende Sacklochbohrung 23.

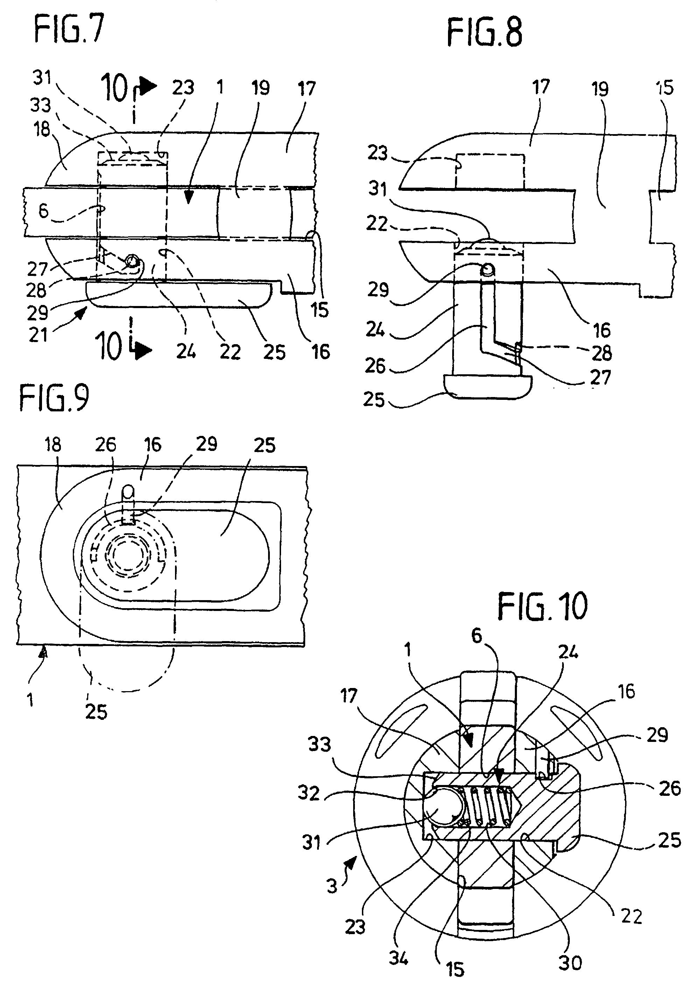

In der Querbohrung 22 ist eine Lagerwelle 24 längsverschieblich und drehbar aufgenommen, die an ihrem aus der Querbohrung 22 nach außen vorstehenden Ende einen radial abstehenden Drehgriff 25 trägt. In den Außenumfang der Lagerwelle 24 ist eine achsparallele Längsnut 26 eingearbeitet, die an ihrem dem Drehgriff 25 benachbarten Ende in Umfangsrichtung umbiegt und dort schräg zur Längsachse der Lagerwelle verläuft (Figur 8). Am Ende dieses schräg verlaufenden Teils 27 der Längsnut 26, der sich über einen Umfangswinkel von etwa 90° erstreckt, weist die Längsnut eine sich in Achsrichtung erstreckende Ausnehmung 28 auf, und zwar springt diese Ausnehmung 28 in Richtung auf das dem Drehgriff 25 abgewandte Ende der Lagerwelle 24 zurück (Figur 7).

In die Längsnut 26 taucht ein Stift 29 ein, der am Arm 16 befestigt ist und radial in die Querbohrung 22 vorsteht. Durch das Eintauchen des Stifts 29 in die Längsnut 26 ist einmal sichergestellt, daß die Lagerwelle 24 in der Querbohrung 22 unverlierbar geführt ist, zum anderen führt eine Drehung der Lagerwelle 24 mit Hilfe des Drehgriffs 25 zu einer axialen Verschiebung der Lagerwelle 24, wenn der Stift 29 sich im schräg verlaufenden Bereich 27 befindet.

In der Lagerwelle 24 ist in einer Sacklochbohrung 30 eine Kugel 31 verschieblich gelagert, die durch eine Öffnung 32 in der Stirnseite 33 der Lagerwelle 24 hervorsteht, die jedoch in der Sacklochbohrung 30 unverlierbar gehalten ist. Durch eine in der Sacklochbohrung 30 angeordnete, sich einerseits an der Kugel 31 und andererseits an der Lagerwelle 24 abstützende Druckfeder 34 wird die Kugel 31 aus der Sacklochbohrung 30 herausgeschoben.

Die Lagerwelle 24 ist an der Querbohrung 22 zwischen zwei Endstellungen verschiebbar. Die eine Endstellung ist die Lagerstellung, in dieser Lagerstellung ist die Lagerwelle 24 maximal in die Querbohrung 22 eingeschoben. Sie taucht dabei in die Sacklochbohrung 23 ein und stützt sich mittels der Kugel 31 federnd am Boden der Sacklochbohrung 23 ab. In dieser Lagerstellung taucht der Stift 29 in die Ausnehmung 28 am Ende des schräg verlaufenden Bereich 27 der Längsnut 26 ein und wird durch die federnde Verschiebung der Lagerwelle 24 in dieser Ausnehmung 28 gehalten. Man erhält auf diese Weise eine Art Bajonettverriegelung der Lagerwelle 24 in der Lagerstellung.

Um diese Stellung lösen zu können, muß die Lagerwelle 24 mit Hilfe des Drehgriffs 25 verdreht werden. Dabei tritt der Stift 29 aus der Ausnehmung 28 aus, muß dazu jedoch die Lagerwelle 24 gegen die Kraft der Druckfeder 34 geringfügig verschieben, das heißt ein Verdrehen der Lagerwelle aus der Lagerstellung heraus benötigt eine bestimmte Mindestkraft, so daß ein unbeabsichtigtes Verdrehen ausgeschlossen ist.

In der anderen Endstellung der Lagerwelle 24, der sogenannten Freigabestellung, ist die Lagerwelle 24 so weit aus der Querbohrung herausgezogen, daß der Stift 29 an den dem schräg verlaufenden Ende der Längsnut 26 gegenüberliegenden Ende anschlägt (Figur 8). Dabei ragt die Kugel 31 der Lagerwelle 24 noch geringfügig in den Zwischenraum 15 vor (Figur 8).

In dieser Freigabestellung kann das Maulteil 1 in den Zwischenraum 15 der beiden Arme 16 und 17 eingesetzt werden, und zwar derart, daß die Lageröffnung 6 mit der Querbohrung 22 und der Sacklochbohrung 23 ausgerichtet wird. Da die Kugel 31 geringfügig in den Zwischenraum 15 vorsteht, muß der Benutzer mit dem Maulteil 1 bei dessen Einsetzen in den Zwischenraum 15 eine gewisse Kraft aufbringen, um die Kugel 31 gegen die Kraft der Druckfeder 34 in die Sacklochbohrung 30 einzuschieben. Sobald die Lageröffnung 6 mit der Querbohrung 22 und der Sacklochbohrung 23 ausgerichtet ist, springt die Kugel 31 unter der Wirkung der Druckfeder 34 elastisch vor und greift geringfügig in die Lageröffnung 6 ein. Der Benutzer spürt also beim Einsetzen des Maulteils 1 auf diese Weise die richtige Positionierung der Lageröffnung 6 in Ausrichtung mit der Querbohrung 22 und der Sacklochbohrung 23 durch das Vorspringen der Kugel 31 und kann danach die Lagerwelle 24 in der Querbohrung 22 vorschieben. Diese durchdringt dabei die Lageröffnung 6 im Maulteil 1 und gelangt schließlich in die Sacklochbohrung 23. Durch Verdrehen des Drehgriffs 25 wird die Lagerwelle 24 in die Lagerstellung vorgeschoben und durch Eingriff des Stifts 29 in die Ausnehmung 28 in der beschriebenen Weise verrastet. Das Maulteil 1 ist auf diese Weise schwenkbar zwischen den Armen 16 und 17 gelagert. Ein Lösen des Maulteils 1 ist in umgekehrter Weise möglich, indem die Lagerwelle 24 mittels des Drehgriffs 25 um 90° verdreht und dann aus der Querbohrung 22 herausgezogen wird.

In der Führungshülse 13 ist ein Kolben 35 längsverschieblich gelagert, an dem sich eine Schraubenfeder 36 abstützt, die anschließend an den Kolben 35 in der Führungshülse 13 angeordnet ist und deren gegenüberliegendes Ende an einem ebenfalls längsverschieblich in der Führungshülse 13 gelagerten Druckteller 37 anliegt. Auf der den Armen 16 und 17 gegenüberliegenden Seite ist die Führungshülse 13 mittels eines eingeschraubten Abschlußstopfens 38 verschlossen, der eine zentrale, längsverlaufende Gewindebohrung 39 aufweist. In diese ist eine kopflose Stellschraube 40 eingeschraubt, die an dem Druckteller 37 anliegt und daher den Abstand des Drucktellers 37 vom Abschlußstopfen 38 entsprechend der jeweiligen Einschraubtiefe bestimmt. Die Gewindebohrung 39 ist durch einen Gewindestopfen 41 verschlossen, der in den freibleibenden Teil der Gewindebohrung 39 eingeschraubt ist.

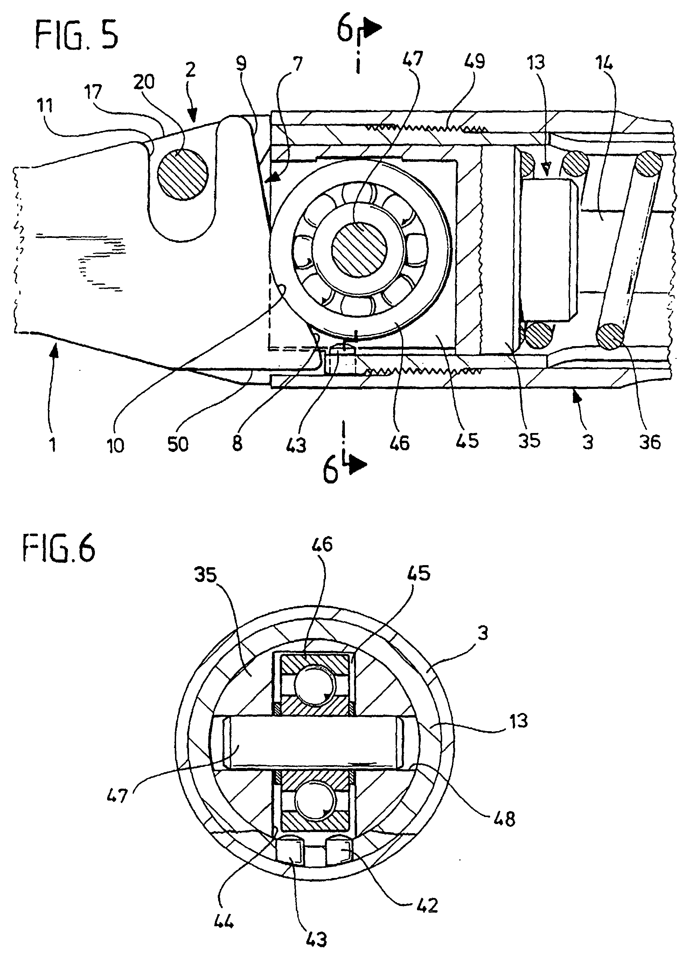

Der Kolben 35 ist in der Führungshülse 13 gegen eine Verdrehung um die Längsachse der Führungshülse 13 dadurch gesichert, daß an der Führungshülse zwei Stifte 42, 43 angeordnet sind, die in einen Längsschlitz 44 des Kolbens 35 hineinragen (Figur 6).

In dem Kolben 35 ist eine Kammer 45 angeordnet, die zu den Armen 16, 17 hin offen ist. In dieser Kammer 45 ist ein Kugellager 46 um eine quer zur Längsachse der Führungshülse 13 verlaufende Drehachse drehbar gelagert und zwar mit Hilfe einer Lagerwelle 47, die in einer Querbohrung 48 des Kolbens 35 angeordnet ist (Figur 6).

Wenn ein Maulteil 1 zwischen die Arme 16 und 17 eingesetzt ist, wird dieses nach der Herstellung der Schwenklagerung 21 in der beschriebenen Weise so verschwenkt, daß das Maulteil 1 vollständig in den Zwischenraum 15 eintaucht, das Maulteil 1 also parallel zu den Armen 16 und 17 ausgerichtet ist. Beim Einschwenken des Maulteils 1 in diese Lage gelangt zunächst der geradlinige Bereich 9 der Stirnkante 7 an dem Kugellager 46 zur Anlage und verschiebt dieses zusammen mit dem es aufnehmenden Kolben 35 gegen die Kraft der Druckfeder 36 geringfügig in der Führungshülse 13, bis das Kugellager 46 in die bogenförmige Vertiefung 10 in der Stirnkante 7 einrastet, wobei der Kolben 35 unter der Wirkung der Druckfeder 36 wieder aus der Führungshülse 13 ausgeschoben wird. In dieser Position, in der das Kugellager 46 in die Vertiefung 10 der Stirnkante 7 eintaucht, befindet sich das Maulteil 1 in der Arbeitsstellung und wird in dieser durch das Eingreifen des Kugellagers 46 in der Vertiefung 10 dann auch gehalten, das heißt der Schwenkwinkel des Maulteils 1 gegenüber dem Griffteil 2 wird auf diese Weise fixiert.

In dieser Arbeitsstellung des Maulteils 1 kann von der Rückseite her die Griffhülse 3 über die Führungshülse 13 des Griffteils 2 geschoben und auf ein Außengewinde 49 der Führungshülse 13 aufgeschraubt werden. Sobald die Griffhülse 3 vollständig auf das Außengewinde 49 aufgeschraubt ist, umgreift sie die untere Kante 50 des Maulteils 1 in geringem Abstand (Figur 5) so daß ein Verschwenken des Maulteils 1 in einer Richtung verhindert wird, nämlich in der Richtung, in der das Maulteil 1 vorher in die Arbeitsstellung verschwenkt worden ist.

In der beschriebenen Positionierung, bei der das Kugellager 46 in die Vertiefung 10 der Stirnkante 7 des Maulteils 1 eingreift und in der die Griffhülse 3 vollständig auf das Außengewinde 49 aufgeschraubt ist, ist das beschriebene Instrument einsatzbereit. Beim Verdrehen des Instruments in einer ersten Richtung, in der dargestellten Zeichnung im Uhrzeigersinn, ergibt sich eine drehfeste Verbindung zwischen Maulteil 1 und Griffteil 2, solange das Kugellager 46 in die Vertiefung 10 eintaucht. Da das Maulteil 1 am Griffteil 2 schwenkbar gelagert ist, wird beim Ausüben eines Drehmoments von der Stirnkante 7 eine Kraft auf das Kugellager 46 und damit den das Kugellager 46 aufnehmenden Kolben 35 ausgeübt, die versucht, das Kugellager 46 aus der Vertiefung auszuheben. Dem wirkt die Kraft der Druckfeder 36 entgegen, die das Kugellager 46 in die Vertiefung 10 hineindrückt.

Bei niedrigen Drehmomenten reicht die Kraft der Feder 36 aus, das Kugellager 46 in der Vertiefung 10 zu halten, eine drehfeste Verbindung zwischen Maulteil 1 und Griffteil 2 bleibt daher aufrechterhalten.

Beim Erreichen eines bestimmten Drehmoments jedoch kann die Druckfeder 36 das Kugellager 46 nicht mehr in der Vertiefung 10 halten, das Kugellager 46 wird entgegen der Kraft der Druckfeder 36 aus der Vertiefung 10 ausgehoben, und damit ist die drehfeste Verbindung zwischen Maulteil 1 und Griffteil 2 aufgehoben. Das Maulteil 1 schwenkt dann um die Schwenklagerung 21, das heißt das Maulteil 1 knickt gegenüber dem Griffteil 2 seitlich aus. Diese Ausknickbewegung wird dadurch begrenzt, daß der in den Ausschnitt 11 eintauchende Querstift 20 an der Kante des Ausschnitts 11 anschlägt. In dieser ausgeknickten Stellung kann der Benutzer daher die Schraubbewegung fortsetzen, und zwar mit einem höheren Drehmoment. Er bemerkt aber ohne weiteres das Ausknicken des Instruments und damit das Überschreiten des maximalen Drehmoments, so daß er darauf hingewiesen wird, daß das jetzt im ausgeknickten Zustand ausgeübte Dreh-moment einen Schwellwert überschreitet.

Bei aus der Vertiefung 10 ausgehobenem Kugellager 46 liegt dieses an dem geradlinigen Bereich 8 der Stirnkante 7 an und übt aufgrund der Kraft der Feder 36 eine Rückstellkraft auf das Maulteil 1 aus, durch die das Maulteil 1 wieder in die Arbeitsstellung zurückverschwenkt wird, in der das Kugellager 46 wieder in die Vertiefung 10 eintauchen kann. Wenn der Benutzer beim Ausknicken des Instruments die Einschraubbewegung beendet, gelangt das Instrument also selbsttätig wieder in die Arbeitsstellung, wenn er allerdings die Einschraubbewegung fortsetzt, bleibt das Instrument im ausgeknickten Zustand und signalisiert dem Benutzer dadurch, daß der Maximalwert des Drehmoments überschritten ist.

Beim Verdrehen des Instruments in der entgegengesetzten Richtung kann keine derartige Ausknickbewegung erfolgen, da das Maulteil 1 durch die Griffhülse 3 an einer Ausknickbewegung gehindert wird. Die Griffhülse 3 wirkt also als Anschlag, der ein Ausknicken des Maulteils 1 nur in einer Richtung ermöglicht.

Die Griffhülse verhindert auch die Entnahme des Maulteils 1 aus dem Griffteil 2, da zur Entnahme das Maulteil 1 entgegen der Ausknickrichtung verschwenkt werden muß, um den Querstift 20 aus dem Ausschnitt 11 des Maulteils 1 herauszubewegen. Wenn also ein Maulteil 1 ausgewechselt werden muß, muß der Benutzer zunächst die Griffhülse 3 auf dem Außengewinde 49 so weit zurückschrauben, bis die untere Kante 50 des Maulteils 1 freigegeben wird. Die Griffhülse kann dabei allerdings auf der Führungshülse 13 verbleiben, da das Außengewinde 49 eine ausreichende Höhe aufweist.

Erst nach diesem Ausschwenken des Maulteils 1 wird die Schwenklagerung 21 in der beschriebenen Weise gelöst, so daß dann gegebenenfalls ein neues Maulteil eingesetzt werden kann.

Zur Reinigung des beschriebenen Instruments wird dieses in der beschriebenen Weise vollständig zerlegt, und zwar in das Maulteil 1, das Griffteil 2 und die Griffhülse 3, die dann getrennt sterilisiert werden. Alle Teile sind dabei frei zugänglich, so daß eine gründliche Reinigung und Sterilisierung erfolgen kann.

## Patentansprüche

1. Chirurgischer Drehmomentschlüssel mit einem Maulteil zur Anlage an einem Drehelement und mit einem an das Maulteil anschließenden Griffteil zum Verdrehen des Maulteils, bei dem das Maulteil (1) und das Griffteil (2) um eine parallel zur Drehachse des Drehelements verlaufende Schwenkachse (21) schwenkbar miteinander verbunden sind, bei dem zwischen Maulteil (1) und Griffteil (2) eine Maulteil (1) und Griffteil (2) in einer bestimmten Winkelstellung relativ zueinander fixierende, beim Überschreiten eines bestimmten Drehmoments lösbare Halteeinrichtung (10, 46) vorgesehen ist und bei dem zwischen Maulteil (1) und Griffteil (2) ein Anschlag (11, 20) wirksam ist, der bei gelöster Halteeinrichtung (10, 46) den Schwenkwinkel des Maulteils (1) gegenüber dem Griffteil (2) begrenzt, **dadurch gekennzeichnet, daß** zwischen Griffteil (2) und Maulteil (1) eine Feder (36) wirksam ist, unter deren Einwirkung das Maulteil (1) aus einer Überlaststellung mit gelöster Halteeinrichtung (10, 46) in die Arbeitsstellung zurückschwenkt, in der die Halteeinrichtung (10,46) aktiv ist.

2. Chirurgischer Drehmomentschlüssel nach Anspruch 1, **dadurch gekennzeichnet, daß** zwischen Maulteil (1) und Griffteil (2) ein Anschlag (3, 50) wirksam ist, der bei gelöster Halteeinrichtung (10, 46) ein Verschwenken des Maulteils (1) gegenüber dem Griffteil (2) nur in einer Richtung ermöglicht.

3. Chirurgischer Drehmomentschlüssel nach einem der voranstehenden Ansprüche, **dadurch gekennzeichnet, daß** die Schwenkachse (21) zwischen dem Maul (5) des Maulteils (1) und dem hinteren Ende (7) des Maulteils (1) angeordnet ist und daß die Halteeinrichtung (10, 46) am hinteren Ende (7) des Maulteils (1) angreift.

4. Chirurgischer Drehmomentschlüssel nach Anspruch 3, **dadurch gekennzeichnet, daß** die Schwenkachse (21) zum Maul (5) einen wesentlich geringeren Abstand aufweist als zum hinteren Ende (7).

5. Chirurgischer Drehmomentschlüssel nach einem der voranstehenden Ansprüche, **dadurch gekennzeichnet, daß** die Halteeinrichtung (10, 46) ein Formgehemme mit fester Auslösekraft ist.

6. Chirurgischer Drehmomentschlüssel nach Anspruch 5, **dadurch gekennzeichnet, daß** die Halteeinrichtung einen in Richtung auf die Schwenkachse (21) verschiebbaren, federbelasteten Rastkörper (46) umfaßt, der in eine Ausnehmung (10) am Maulteil (1) eintaucht und beim Überschreiten eines bestimmten Drehmoments gegen die Federbelastung aus der Ausnehmung (10) ausgehoben wird.

7. Chirurgischer Drehmomentschlüssel nach Anspruch 6, **dadurch gekennzeichnet, daß** der Rastkörper (46) im Griffteil (2) längsverschieblich gelagert ist.

8. Chirurgischer Drehmomentschlüssel nach Anspruch 7, **dadurch gekennzeichnet, daß** der Rastkörper (46) in einem im Griffteil (2) längsverschieblichen Führungskörper (35) gelagert ist, an dem eine am Griffteil (2) abgestützte Feder (36) angreift.

9. Chirurgischer Drehmomentschlüssel nach Anspruch 8, **dadurch gekennzeichnet, daß** das Griffteil (2) eine zylindrische Führungshülse (13) umfaßt, in der ein kolbenförmiger Führungskörper (35) längsverschieblich gelagert ist und die eine Druckfeder (36) aufnimmt.

10. Chirurgischer Drehmomentschlüssel nach Anspruch 9, **dadurch gekennzeichnet, daß** der Führungskörper (35) und seine Führung (13) eine schmiermittelfreie Kunststoff-Metall-Paarung bilden.

11. Chirurgischer Drehmomentschlüssel nach Anspruch 10, **dadurch gekennzeichnet, daß** der Führungskörper (35) aus Polyetheretherketon und die Führung (13) aus rostfreiem Stahl bestehen.

12. Chirurgischer Drehmomentschlüssel nach einem der Ansprüche 9 bis 11, **dadurch gekennzeichnet, daß** der Führungskörper (35) in der Führungshülse (13) gegen eine Verdrehung um die Längsachse der Führungshülse (13) gesichert ist.

13. Chirurgischer Drehmomentschlüssel nach einem der Ansprüche 6 bis 12, **dadurch gekennzeichnet, daß** die Federbelastung des Rastkörpers (46) einstellbar ist.

14. Chirurgischer Drehmomentschlüssel nach Anspruch 13, **dadurch gekennzeichnet, daß** zur Einstellung der Federbelastung die Abstützung (37) der Feder (36) verstellbar ist.

15. Chirurgischer Drehmomentschlüssel nach einem der Ansprüche 9 bis 12 und 13, **dadurch gekennzeichnet, daß** die Führungshülse (13) einen Abschlußstopfen (38) aufweist, an dem ein verstellbarer Abstandshalter (40) für eine Druckfederabstützung (37) angeordnet ist.

16. Chirurgischer Drehmomentschlüssel nach Anspruch 15, **dadurch gekennzeichnet, daß** der Abstandshalter eine Stellschraube (40) umfaßt, die an einem in der Führungshülse (13) längsverschieblichen Druckteller (37) anliegt.

17. Chirurgischer Drehmomentschlüssel nach Anspruch 16, **dadurch gekennzeichnet, daß** die Stellschraube (40) in einer Gewindebohrung (39) des Abschlußstopfens (38) versenkt angeordnet ist und daß die Gewindebohrung (39) durch einen in diese eingesetzten Stopfen (41) verschlossen ist.

18. Chirurgischer Drehmomentschlüssel nach Anspruch 17, **dadurch gekennzeichnet, daß** der Stopfen ein Gewindestopfen (41) ist.

19. Chirurgischer Drehmomentschlüssel nach einem der Ansprüche 9 bis 18, **dadurch gekennzeichnet, daß** die Führungshülse (13) seitliche Durchbrechungen (14) aufweist.

20. Chirurgischer Drehmomentschlüssel nach einem der Ansprüche 9 bis 19, **dadurch gekennzeichnet, daß** über die Führungshülse (13) eine lösbar mit dieser verbundene Griffhülse (3) geschoben ist.

21. Chirurgischer Drehmomentschlüssel nach Anspruch 20, **dadurch gekennzeichnet, daß** die Griffhülse (3) auf die Führungshülse (13) aufgeschraubt ist.

22. Chirurgischer Drehmomentschlüssel nach einem der Ansprüche 20 oder 21, **dadurch gekennzeichnet, daß** die Griffhülse (3) die Führungshülse (13) und einen in sie eingesetzten Abschlußstopfen (38) überdeckt.

23. Chirurgischer Drehmomentschlüssel nach einem der Ansprüche 20 bis 22, **dadurch gekennzeichnet, daß** die Griffhülse (3) in ihrer vollständig auf die Führungshülse (13) aufgeschobenen Position den Anschlag für das Maulteil (1) bildet, der bei gelöster Halteeinrichtung (10, 46) ein Verschwenken des Maulteils (1) gegenüber dem Griffteil (2) nur in einer Richtung ermöglicht.

24. Chirurgischer Drehmomentschlüssel nach einem der Ansprüche 6 bis 23, **dadurch gekennzeichnet, daß** der Rastkörper eine parallel zur Schwenkachse (21) des Maulteils (1) drehbare Rolle (46) ist.

25. Chirurgischer Drehmomentschlüssel nach Anspruch 24, **dadurch gekennzeichnet, daß** die Rolle ein Kugellager (46) ist.

26. Chirurgischer Drehmomentschlüssel nach Anspruch 25, **dadurch gekennzeichnet, daß** die Rolle ein gedichtetes Kugellager (46) ist, das mit einem sterilisierbaren Fett gefüllt ist.

27. Chirurgischer Drehmomentschlüssel nach einem der Ansprüche 6 bis 26, **dadurch gekennzeichnet, daß** die Ausnehmung am Maulteil (1) eine bogenförmige Vertiefung (10) in einer hinteren Stirnkante (7) des Maulteils (1) ist.

28. Chirurgischer Drehmomentschlüssel nach Anspruch 27, **dadurch gekennzeichnet, daß** die Stirnkante (7) gegenüber einer Tangente an einen um die Schwenkachse (21) des Maulteils (1) gelegten Kreis geringfügig geneigt ist.

29. Chirurgischer Drehmomentschlüssel nach einem der voranstehenden Ansprüche, **dadurch gekennzeichnet, daß** das Griffteil (2) zwei parallele Arme (16, 17) aufweist, zwischen denen das Maulteil (1) aufgenommen ist, und daß die Schwenkachse (21) des Maulteils (1) am vorderen Ende (18) der Arme (16, 17) angeordnet ist.

30. Chirurgischer Drehmomentschlüssel nach Anspruch 29, **dadurch gekennzeichnet, daß** ein die beiden Arme (16, 17) verbindender Steg (20) den Anschlag bildet, der bei gelöster Halteeinrichtung (10, 46) den Schwenkwinkel des Maulteils (1) gegenüber dem Griffteil (2) beschränkt.

31. Chirurgischer Drehmomentschlüssel nach einem der voranstehenden Ansprüche, **dadurch gekennzeichnet, daß** die Schwenklagerung (21) des Maulteils (1) am Griffteil (2) lösbar ist.

32. Chirurgischer Drehmomentschlüssel nach Anspruch 31, **dadurch gekennzeichnet, daß** am Griffteil (2) eine Lagerwelle (24) axial verschiebbar angeordnet ist zwischen einer Lagerstellung, in der sie eine Lageröffnung (6) des Maulteils (1) durchsetzt, und einer Freigabestellung, in der sie aus der Lageröffnung (6) herausgezogen ist.

33. Chirurgischer Drehmomentschlüssel nach Anspruch 32, **dadurch gekennzeichnet, daß** die Lagerwelle (24) unverlierbar an dem Griffteil (2) gehalten ist.

34. Chirurgischer Drehmomentschlüssel nach Anspruch 33, **dadurch gekennzeichnet, daß** am Griffteil (2) ein Vorsprung (29) angeordnet ist, der in eine Längsnut (26) der Lagerwelle (24) eintaucht.

35. Chirurgischer Drehmomentschlüssel nach Anspruch 34, **dadurch gekennzeichnet, daß** die Längsnut (26) der Lagerwelle (24) einen in Umfangsrichtung umbiegenden, schräg zur Längsachse der Lagerwelle (24) verlaufenden Endbereich (27) aufweist und daß an der Lagerwelle (24) ein Drehgriff (25) angeordnet ist.

36. Chirurgischer Drehmomentschlüssel nach Anspruch 35, **dadurch gekennzeichnet, daß** am Ende des schräg verlaufenden Endbereichs (27) der Längsnut (26) eine in Längsrichtung der Lagerwelle (24) rückspringende, den Vorsprung (29) am Griffteil (2) in der Lagerstellung der Lagerwelle (24) aufnehmende Ausnehmung (28) in der Längsnut (26) angeordnet ist und daß die Lagerwelle (24) in der Lagerstellung federnd gegen den Vorsprung (29) am Griffteil (2) gedrückt ist.

37. Chirurgischer Drehmomentschlüssel nach Anspruch 36, **dadurch gekennzeichnet, daß** in der Lagerwelle (24) ein stirnseitig aus dieser hervorstehender, federnd in diese eindrückbarer Druckkörper (31) gelagert ist.

38. Chirurgischer Drehmomentschlüssel nach Anspruch 37, **dadurch gekennzeichnet, daß** der Druckkörper eine Kugel (31) ist.

39. Chirurgischer Drehmomentschlüssel nach Anspruch 37 oder 38, **dadurch gekennzeichnet, daß** der Druckkörper (31) in der Freigabestellung der Lagerwelle (24) in den Einschubraum (15) des Maulteils (1) hineinragt.

## Claims

1. A surgical torque wrench comprising a jaw part for application to a rotary component, and a gripping part connected to the jaw part for rotation thereof, wherein the jaw part (1) and the gripping part (2) are connected to one another so as to be pivotable about a pivoting axis (21) extending parallel to the rotation axis of the rotary component, wherein a retaining device (10, 46), fixing a jaw part (1) and a gripping part (2) in a particular angular position relative to one another and releasable when a particular turning moment is exceeded, is provided between the jaw part (1) and the gripping part (2), and wherein a stop (11, 20) is effective between the jaw part (1) and the gripping part (2) and limits the pivoting angle of the jaw part (1) relative to the gripping part (2) when the retaining device (10, 46) is released, **characterised in that** a spring (36) is effective between the gripping part (2) and the jaw part (1), under the action of which the jaw part (1) pivots back from an overload position, in which the retaining device (10, 46) is released, into the operating position in which the retaining device (10, 46) is active.

2. A surgical torque wrench according to claim 1, **characterised in that** a stop (3, 50) is effective between the jaw part (1) and the gripping part (2) and, when the retaining device (10, 46) is released, permits pivoting of the jaw part (1) relative to the gripping part (2) in one direction only.

3. A surgical torque wrench according to any one of the preceding claims, **characterised in that** the pivoting axis (21) is arranged between the jaw (5) of the jaw part (1) and the rear end (7) of the jaw part (1), and **in that** the retaining device (10, 46) engages the rear end (7) of the jaw part (1).

4. A surgical torque wrench according to claim 3, **characterised in that** the pivoting axis (21) is at a substantially shorter distance from the jaw (5) than from the rear end (7).

5. A surgical torque wrench according to any one of the preceding claims, **characterised in that** the retaining device (10, 46) is a positive-locking device with a fixed release force.

6. A surgical torque wrench according to claim 5, **characterised in that** the retaining device comprises a spring-loaded locking member (46) which is displaceable towards the pivoting axis (21), extends into a recess (10) in the jaw part (1) and, when a particular turning moment is exceeded, is lifted out of the recess (10) against the spring loading.

7. A surgical torque wrench according to claim 6, **characterised in that** the locking member (46) is longitudinally displaceably mounted in the gripping part (2).

8. A surgical torque wrench according to claim 7, **characterised in that** the locking member (46) is mounted in a guide member (35) which is longitudinally displaceable in the gripping part (2) and which is engaged by a spring (36) supported on the gripping part (2).

9. A surgical torque wrench according to claim 8, **characterised in that** the gripping part (2) comprises a cylindrical guide sleeve (13), in which a piston-type guide member (35) is longitudinally displaceably mounted and which holds a compression spring (36).

10. A surgical torque wrench according to claim 9, **characterised in that** the guide member (35) and its guide (13) form a lubricant-free plastics/metal pairing.

11. A surgical torque wrench according to claim 10, **characterised in that** the guide member (35) comprises polyetheretherketone and the guide (13) comprises stainless steel.

12. A surgical torque wrench according to any one of claims 9 to 11, **characterised in that** the guide member (35) is secured in the guide sleeve (13) against rotation about the longitudinal axis thereof.

13. A surgical torque wrench according to any one of claims 6 to 12, **characterised in that** the spring loading of the locking member (46) is adjustable.

14. A surgical torque wrench according to claim 13, **characterised in that**, to adjust the spring loading, the support (37) of the spring (36) is adjustable.

15. A surgical torque wrench according to any one of claims 9 to 12 and 13, **characterised in that** the guide sleeve (13) has a closing stopper (38), on which an adjustable spacer (40) for a compression-spring support (37) is arranged.

16. A surgical torque wrench according to claim 15, **characterised in that** the spacer comprises an adjusting screw (40) resting against a pressure plate (37) longitudinally displaceable in the guide sleeve (13).

17. A surgical torque wrench according to claim 16, **characterised in that** the adjusting screw (40) is sunk into a threaded bore (39) of the closing stopper (38) and **in that** the threaded bore (39) is closed by a stopper (41) inserted into it.

18. A surgical torque wrench according to claim 17, **characterised in that** the stopper is a threaded stopper (41).

19. A surgical torque wrench according to any one of claims 9 to 18, **characterised in that** the guide sleeve (13) has lateral openings (14).

20. A surgical torque wrench according to any one of claims 9 to 19, **characterised in that** a gripping sleeve (3) is pushed over the guide sleeve (13) and is detachably connected thereto.

21. A surgical torque wrench according to claim 20, **characterised in that** the gripping sleeve (3) is screwed onto the guide sleeve (13).

22. A surgical torque wrench according to either one of claims 20 and 21, **characterised in that** the gripping sleeve (3) covers the guide sleeve (13) and a closing stopper (38) inserted into it.

23. A surgical torque wrench according to any one of claims 20 to 22, **characterised in that**, when the gripping sleeve (3) is fully pushed onto the guide sleeve (13), it forms the stop for the jaw part (1), the stop permitting pivoting of the jaw part (1) relative to the gripping part (2) in one direction only when the retaining device (10, 46) is released.

24. A surgical torque wrench according to any one of claims 6 to 23, **characterised in that** the locking member is a roller (46) rotatable parallel to the pivoting axis (21) of the jaw part (1).

25. A surgical torque wrench according to claim 24, **characterised in that** the roller is a ball bearing (46).

26. A surgical torque wrench according to claim 25, **characterised in that** the roller is a sealed ball bearing (46) filled with a sterilisable grease.

27. A surgical torque wrench according to any one of claims 6 to 26, **characterised in that** the recess in the jaw part (1) is a curved depression (10) in a rear end surface (7) of the jaw part (1).

28. A surgical torque wrench according to claim 27, **characterised in that** the end surface (7) is slightly inclined relative to a tangent to a circle round the pivoting axis (21) of the jaw part (1).

29. A surgical torque wrench according to any one of the preceding claims, **characterised in that** the gripping part (2) has two parallel arms (16, 17), between which the jaw part (1) is held, and **in that** the pivoting axis (21) of the jaw part (1) is arranged at the front end (18) of the arms (16, 17).

30. A surgical torque wrench according to claim 29, **characterised in that** a crosspiece (20) connecting the two arms (16, 17) forms the stop which limits the pivoting angle of the jaw part (1) relative to the gripping part (2) when the retaining device (10, 46) is released.

31. A surgical torque wrench according to any one of the preceding claims, **characterised in that** the pivoted mounting (21) of the jaw part (1) on the gripping part (2) is disconnectable.

32. A surgical torque wrench according to claim 31, **characterised in that** a bearing shaft (24) is axially displaceably arranged on the gripping part (2) between a bearing position, in which it extends through a bearing opening (6) in the jaw part (1), and a release position in which it is withdrawn from the bearing opening (6).

33. A surgical torque wrench according to claim 32, **characterised in that** the bearing shaft (24) is captively held on the gripping part (2).

34. A surgical torque wrench according to claim 33, **characterised in that** a projection (29), extending into a longitudinal groove (26) of the bearing shaft (24), is arranged on the gripping part (2).

35. A surgical torque wrench according to claim 34, **characterised in that** the longitudinal groove (26) of the bearing shaft (24) has an end region (27) bending round in the circumferential direction and extending obliquely to the longitudinal axis of the bearing shaft (24), and **in that** a rotary grip (25) is arranged on the bearing shaft (24).

36. A surgical torque wrench according to claim 35, **characterised in that**, at the end of the obliquely extending end region (27) of the longitudinal groove (26), a recess (28), returning in the longitudinal direction of the bearing shaft (24) and receiving the projection (29) on the gripping part (2) when the bearing shaft (24) is in the bearing position, is arranged in the longitudinal groove (26), and **in that**, when the bearing shaft (24) is in the bearing position, it is pressed resiliently against the projection (29) on the gripping part (2).

37. A surgical torque wrench according to claim 36, **characterised in that** a pressure member (31) projecting from the end of the bearing shaft (24) is resiliently pushable into the latter and is mounted therein.

38. A surgical torque wrench according to claim 37, **characterised in that** the pressure member is a ball (31).

39. A surgical torque wrench according to claim 37 or 38, **characterised in that** the pressure member (31) projects into the insertion space (15) of the jaw part (1) when the bearing shaft (24) is in the release position.

## Revendications

1. Clé dynamométrique à usage chirurgical avec une partie formant mâchoire à appliquer sur un élément rotatif et avec une partie formant poignée se raccordant à la partie formant mâchoire pour faire tourner la partie formant mâchoire, selon laquelle la partie formant mâchoire (1) et la partie formant poignée (2) sont mutuellement reliées à pivotement autour d'un axe de pivotement (21) parallèle à l'axe de rotation de l'élément rotatif, selon laquelle il est prévu entre la partie formant mâchoire (1) et la partie formant poignée (2) un dispositif de retenue (10, 46) immobilisant dans une position angulaire relative donnée la partie formant mâchoire (1) et la partie formant poignée (2) et libérable en cas de dépassement d'un couple de rotation donné, et selon laquelle une butée (11, 20) est active entre la partie formant mâchoire (1) et la partie formant poignée (2), butée qui, lorsque le dispositif de retenue (10, 46) est libéré, limite l'angle de pivotement de la partie formant mâchoire (1) par rapport à la partie formant poignée (2), **caractérisée en ce qu'**un ressort (36) est actif entre la partie formant poignée (2) et la partie formant mâchoire (1), sous l'action duquel la partie formant mâchoire (1) pivote en retour d'une position de surcharge avec le dispositif de retenue (10, 46) libéré dans la position de travail avec le dispositif de retenue (10, 46) actif.

2. Clé dynamométrique à usage chirurgical selon la revendication 1, **caractérisée en ce qu'**une butée (3, 50) est active entre la partie formant mâchoire (1) et la partie formant poignée (2), butée qui, lorsque le dispositif de retenue (10, 46) est libéré, n'autorise un pivotement de la partie formant mâchoire (1) par rapport à la partie formant poignée (2) que dans une direction.

3. Clé dynamométrique à usage chirurgical selon l'une des revendications précédentes, **caractérisée en ce que** l'axe de pivotement (21) est disposé entre la mâchoire (5) de la partie formant mâchoire (1) et l'extrémité arrière (7) de la partie formant mâchoire (1), et **en ce que** le dispositif de retenue (10, 46) agit sur l'extrémité arrière (7) de la partie formant mâchoire (1).

4. Clé dynamométrique à usage chirurgical selon la revendication 3, **caractérisée en ce que** l'axe de pivotement (21) est nettement plus proche de la mâchoire (5) que de l'extrémité arrière (7).

5. Clé dynamométrique à usage chirurgical selon l'une des revendications précédentes, **caractérisée en ce que** le dispositif de retenue (10, 46) est un dispositif de verrouillage par complémentarité de forme avec une force de libération fixe.

6. Clé dynamométrique à usage chirurgical selon la revendication 5, **caractérisée en ce que** le dispositif de retenue comprend un élément d'enclenchement (46) sollicité par ressort qui peut être déplacé en direction de l'axe de pivotement (21), et qui s'enfonce dans un évidement (10) sur la partie formant mâchoire (1) et est soulevé hors de l'évidement (10), contre la charge de ressort, en cas de dépassement d'un couple de rotation donné.

7. Clé dynamométrique à usage chirurgical selon la revendication 6, **caractérisée en ce que** l'élément d'enclenchement (46) est monté à déplacement longitudinal dans la partie formant poignée (2).

8. Clé dynamométrique à usage chirurgical selon la revendication 7, **caractérisée en ce que** l'élément d'enclenchement (46) est monté dans un corps de guidage (35) pouvant être déplacé longitudinalement dans la partie formant poignée (2) et sur lequel agit un ressort (36) qui s'appuie sur la partie formant poignée (2).

9. Clé dynamométrique à usage chirurgical selon la revendication 8, **caractérisée en ce que** la partie formant poignée (2) comprend un manchon de guidage cylindrique (13), dans lequel un corps de guidage (35) en forme de piston est monté à déplacement longitudinal et qui reçoit un ressort de pression (36).

10. Clé dynamométrique à usage chirurgical selon la revendication 9, **caractérisée en ce que** le corps de guidage (35) et son guide (13) forment un appariement plastique-métal sans lubrifiant.

11. Clé dynamométrique à usage chirurgical selon la revendication 10, **caractérisée en ce que** le corps de guidage (35) est réalisé en polyétheréthercétone et le guide (13) en acier inoxydable.

12. Clé dynamométrique à usage chirurgical selon l'une des revendications 9 à 11, **caractérisée en ce que** le corps de guidage (35) est bloqué dans le manchon de guidage (13) en rotation autour de l'axe longitudinal du manchon de guidage (13).

13. Clé dynamométrique à usage chirurgical selon l'une des revendications 6 à 12, **caractérisée en ce que** la charge de ressort de l'élément d'enclenchement (46) est réglable.

14. Clé dynamométrique à usage chirurgical selon la revendication 13, **caractérisée en ce que**, afin de régler la charge de ressort, le support (37) du ressort (36) est réglable.

15. Clé dynamométrique à usage chirurgical selon l'une des revendications 9 à 12 et 13, **caractérisée en ce que** le manchon de guidage (13) présente un bouchon de fermeture (38), sur lequel est disposé un écarteur réglable (40) pour un support (37) de ressort de pression.

16. Clé dynamométrique à usage chirurgical selon la revendication 15, **caractérisée en ce que** l'écarteur consiste en une vis de réglage (40) qui s'applique contre une plaque de pression (37) pouvant être déplacée longitudinalement dans le manchon de guidage (13).

17. Clé dynamométrique à usage chirurgical selon la revendication 16, **caractérisée en ce que** la vis de réglage (40) est disposée noyée dans un perçage fileté (39) du bouchon de fermeture (38), et **en ce que** le perçage fileté (39) est fermé par un bouchon (41) inséré dans ce perçage.

18. Clé dynamométrique à usage chirurgical selon la revendication 17, **caractérisée en ce que** le bouchon est un bouchon fileté (41).

19. Clé dynamométrique à usage chirurgical selon l'une des revendications 9 à 18, **caractérisée en ce que** le manchon de guidage (13) présente des ouvertures latérales (14).

20. Clé dynamométrique à usage chirurgical selon l'une des revendications 9 à 19, **caractérisée en ce qu'**un manchon de poignée (3) est enfilé sur le manchon de guidage (13) en lui étant assemblé de manière amovible.

21. Clé dynamométrique à usage chirurgical selon la revendication 20, **caractérisée en ce que** le manchon de poignée (3) est vissé sur le manchon de guidage (13).

22. Clé dynamométrique à usage chirurgical selon la revendication 20 ou 21, **caractérisée en ce que** le manchon de poignée (3) recouvre le manchon de guidage (13) et un bouchon de fermeture (38) inséré dans ce dernier.

23. Clé dynamométrique à usage chirurgical selon l'une des revendications 20 à 22, **caractérisée en ce que** le manchon de poignée (3), dans sa position totalement enfilée sur le manchon de guidage (13), constitue la butée pour la partie formant mâchoire (1) qui autorise, lorsque le dispositif de retenue (10, 46) est libéré, un pivotement dans une seule direction de la partie formant mâchoire (1) par rapport à la partie formant poignée (2).

24. Clé dynamométrique à usage chirurgical selon l'une des revendications 6 à 23, **caractérisée en ce que** l'élément d'enclenchement est un galet de roulement (46) rotatif parallèlement à l'axe de pivotement (21) de la partie formant mâchoire (1).

25. Clé dynamométrique à usage chirurgical selon la revendication 24, **caractérisée en ce que** le galet de roulement est un roulement à billes (46).

26. Clé dynamométrique à usage chirurgical selon la revendication 25, **caractérisée en ce que** le galet de roulement est un roulement à billes garni, qui est rempli d'une graisse pouvant être stérilisée.

27. Clé dynamométrique à usage chirurgical selon l'une des revendications 6 à 26, **caractérisée en ce que** l'évidement sur la partie formant mâchoire (1) est un renfoncement arqué (10) dans un bord terminal arrière (7) de la partie formant mâchoire (1).

28. Clé dynamométrique à usage chirurgical selon la revendication 27, **caractérisée en ce que** le bord terminal (7) est légèrement incliné par rapport à une tangente à un cercle tracé autour de l'axe de pivotement (21) de la partie formant mâchoire (1).

29. Clé dynamométrique à usage chirurgical selon l'une des revendications précédentes, **caractérisée en ce que** la partie formant poignée (2) présente deux bras parallèles (16, 17) entre lesquels est reçue la partie formant mâchoire (1), et **en ce que** l'axe de pivotement (21) de la partie formant mâchoire (1) est disposé à l'extrémité avant (18) des bras (16, 17).

30. Clé dynamométrique à usage chirurgical selon la revendication 29, **caractérisée en ce qu'**une entretoise (20) reliant les deux bras (16, 17) constitue la butée qui, lorsque le dispositif de retenue (10, 46) est libéré, limite l'angle de pivotement de la partie formant mâchoire (1) par rapport à la partie formant poignée (2).

31. Clé dynamométrique à usage chirurgical selon l'une des revendications précédentes, **caractérisée en ce que** le palier de pivotement (21) de la partie formant mâchoire (1) sur la partie formant poignée (2) est amovible.

32. Clé dynamométrique à usage chirurgical selon la revendication 31, **caractérisée en ce qu'**un arbre de palier (24) est disposé sur la partie formant poignée (2) à déplacement axial entre une position de montage, dans laquelle il traverse une ouverture de palier (6) de la partie formant mâchoire (1), et une position de libération dans laquelle il est retiré de l'ouverture de palier (6).

33. Clé dynamométrique à usage chirurgical selon la revendication 32, **caractérisée en ce que** l'arbre de palier (24) est retenu de manière imperdable sur la partie formant poignée (2).

34. Clé dynamométrique à usage chirurgical selon la revendication 33, **caractérisée en ce qu'**une saillie (29) est disposée sur la partie formant poignée (2) et s'enfonce dans une rainure longitudinale (26) de l'arbre de palier (24).

35. Clé dynamométrique à usage chirurgical selon la revendication 34, **caractérisée en ce que** la rainure longitudinale (26) de l'arbre de palier (24) présente une région terminale (27) incurvée en direction circonférentielle et s'étendant en oblique par rapport à l'axe longitudinal de l'arbre de palier (24), et **en ce qu'**une poignée tournante (25) est disposée sur l'arbre de palier (24).

36. Clé dynamométrique à usage chirurgical selon la revendication 35, **caractérisée en ce qu'**un évidement (28) est ménagé dans la rainure longitudinale (26) à l'extrémité de la région terminale oblique (27) de la rainure longitudinale (26), évidement qui s'étend en retour dans la direction longitudinale de l'arbre de palier (24) et qui reçoit, dans la position de montage de l'arbre de palier (24), la saillie (29) sur la partie formant poignée (2), et **en ce que**, dans la position de montage, l'arbre de palier (24) est pressé élastiquement contre la saillie (29) sur la partie formant poignée (2).

37. Clé dynamométrique à usage chirurgical selon la revendication 36, **caractérisée en ce qu'**un élément de pression (31) est monté dans l'arbre de palier (24), élément qui dépasse d'une face terminale de l'arbre de palier (24) et peut être enfoncé élastiquement dans l'arbre de palier (24).

38. Clé dynamométrique à usage chirurgical selon la revendication 37, **caractérisée en ce que** l'élément de pression est une bille (31).

39. Clé dynamométrique à usage chirurgical selon la revendication 37 ou 38, **caractérisée en ce que** l'élément de pression (31), dans la position de libération de l'arbre de palier (24), dépasse dans l'espace (15) d'insertion de la partie formant mâchoire (1).
